(19) Europäisches Patentamt
European Patent Office
Office européen des brevets

(11) Veröffentlichungsnummer: **0 111 935**
**B1**

(12) # EUROPÄISCHE PATENTSCHRIFT

(45) Veröffentlichungstag der Patentschrift: **12.10.88**

(51) Int. Cl.⁴: **C 07 D 501/46,** C 07 D 501/57, A 61 K 31/545

(21) Anmeldenummer: **83112860.8**

(22) Anmeldetag: **21.12.83**

(54) **Cephalosporinderivate und Verfahren zu ihrer Herstellung.**

(30) Priorität: **23.12.82 DE 3247614**

(43) Veröffentlichungstag der Anmeldung: **27.06.84 Patentblatt 84/26**

(45) Bekanntmachung des Hinweises auf die Patenterteilung: **12.10.88 Patentblatt 88/41**

(84) Benannte Vertragsstaaten: **AT BE CH DE FR GB IT LI LU NL SE**

(56) Entgegenhaltungen:
**FR-A-2 348 218**
**FR-A-2 385 722**
**GB-A-2 105 334**
**GB-A-2 105 335**
**GB-A-2 106 905**
**US-A-4 278 793**
**US-A-4 450 270**

**Merk-Index 10 Ed., S. 271, Nr.1913(1983)**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Lattrell, Rudolf, Dr., Heuhohlweg 6h, D-6240 Königstein/Taunus (DE)**
Erfinder: **Dürckheimer, Walter, Dr., Im Lerchenfeld 45, D-6234 Hattersheim am Main (DE)**
Erfinder: **Kirrstetter, Reiner, Dr., Am Flachsland 18, D-6233 Kelkheim (Taunus) (DE)**
Erfinder: **Schwab, Wilfried, Dr., Am Flachsland 18, D-6233 Kelkheim (Taunus) (DE)**
Erfinder: **Kiesel, Norbert, Dr., Bornstrasse 50, D-6238 Hofheim am Taunus (DE)**

**EP 0 111 935 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Cephalosporinderivate und Verfahren zu ihrer Herstellung, insbesondere polare Cephemderivate, die in 3-Stellung des Cephemrings durch bestimmte Chinolinium- und Isochinoliniumnethyl-Reste substituiert sind, eine sehr gute antimikrobielle Wirkung gegen grampositive und gramnegative Bakterien besitzen und deshalb als Arzneimittel zur Behandlung von mikrobiellen Infektionen geeignet sind.

Gegenstand der Erfindung sind daher Cephemderivate der allgemeinen Formel I

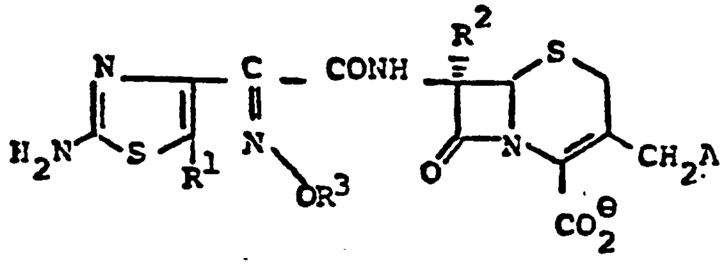

(I)

und deren physiologisch verträglichen Säureadditionssalze, worin bedeuten

$R^1$ Wasserstoff
$R^2$ Wasserstoff,
$R^3$ gegebenenfalls durch Fluor substituiertes $C_1$-$C_4$-Alkyl,
A einen Chinolinium oder einen Isochinoliniumrest, der jeweils auch 1- bis 2-fach substituiert sein kann durch $C_1$-$C_4$-Alkyl, und in der die $R^3O$-Gruppe in syn-Position steht.

Als besonders bevorzugt kommen beispielsweise die folgenden Substituenten in Betracht:

$R^1$: Wasserstoff,
$R^2$: Wasserstoff,
$R^3$: $C_1$-$C_4$-Alkyl, wie z. B. Methyl, Äthyl, Propyl, Isopropyl, Butyl, Isobutyl, Tert.Butyl, insbesondere Methyl, Äthyl, das durch Fluor substituiert ist, insbesondere Trifluoräthyl oder 2,2,3,3-Tetrafluorpropyl;
A : ein Chinoliniumrest

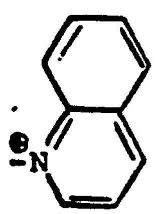

,

der 1- bis 2-fach substituiert sein kann durch $C_1$-$C_4$-Alkyl, wie insbesondere Methyl, Äthyl, Propyl, Isopropyl oder Butyl, oder ein Isochinoliniumrest

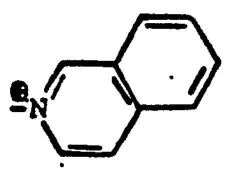

,

der wie vorstehend der Chinoliniumrest substiutiert sein kann. Bevorzugte Beispiele für A sind beispielsweise die folgenden: Chinolinium, 2,3,4,5,6,7-oder 8-Methylchinolinium, Isochinolinium, 1-, 3-, 4-, 5-, 6-, 7- oder 8-Methylisochinolinium.

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man

a) eine Verbindung der allgemeinen Formel II

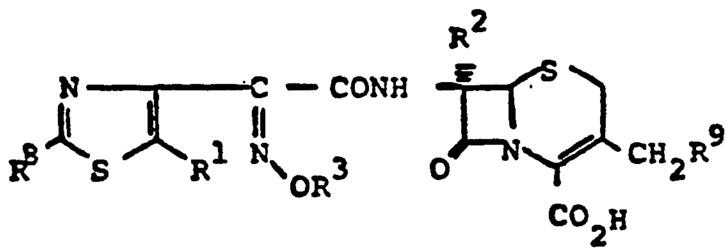

II

oder deren Salze oder ein reaktionsfähiges Derivat der Verbindung II worin $R^1$, $R^2$ und $R^3$ die obengenannte Bedeutung haben und $R^8$ eine Amino- oder eine geschützte Aminogruppe bedeutet und $R^9$ eine durch Chinolin, Isochinolin oder substiutierte Chinoline oder Isochinoline, die den Resten A der Formel I entsprechen, austauschbare Gruppe bedeutet, mit Chinolin, Isochinolin oder einem ihrer Derivate

umsetzt und

α) eine gegebenenfalls vorhandene Schutzgruppe abspaltet und
β) falls erforderlich, das erhaltene Produkt in ein physiologisch verträgliches Säureadditionssalz überführt,
oder
b) eine 7-aminocephemverbindung der allgemeinen Formel

III

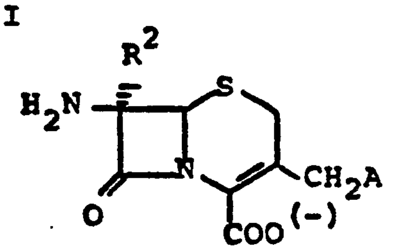

(III)

oder deren Säureadditionssalze, worin $R^2$ und A die obengenannte Bedeutung haben, wobei die Aminogruppe auch in Form eines reaktionsfähigen Derivats vorliegen kann, mit einer 2-(2-Aminothiazol-4-yl)-2-syn-oximinoessigsäure der allgemeinen Formel IV,

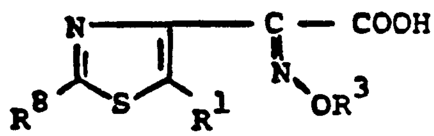

IV

worin $R^1$, $R^3$ und $R^8$ die obige Bedeutung besitzen, oder mit einem aktivierten Derivat dieser Verbindung umsetzt und

α) eine gegebenenfalls vorhandene Schutzgruppe abspaltet und
β) falls erforderlich, das erhaltene Produkt in ein physiologisch verträgliches Säureadditionssalz überführt.

Soll die Herstellung der Verbindungen der allgemeinen Formel I durch nucleophilen Austausch von $R^9$ in den Verbindungen der allgemeinen Formel II durch Chinolin, Isochinolin oder eines ihrer angegebenen Derivate erfolgen, so kommen als Reste $R^8$ insbesondere in Betracht Acyloxyreste von niederen aliphatischen Carbonsäuren vorzugsweise mit 1 bis 4 C-Atomen, wie z. B. Acetoxy oder Propionyloxy, insbesondere Acetoxy, die gegebenenfalls substituiert sein können, wie z. B. Chloracetoxy oder Acetylacetoxy. Für $R^9$ kommen auch andere Gruppen in Betracht, wie beispielsweise Halogen, insbesondere Chlor, Brom oder Jod, oder Carbamoyloxy.

Erfindungsgemäß werden bei der nucleophilen Austauschreaktion Ausgangsverbindungen der allgemeinen Formel II, in denen $R^9$ für Acetoxy steht, eingesetzt, oder deren Salze, wie z. B. ein Natrium- oder Kaliumsalz. Die Reaktion wird in einem Lösungsmittel, vorzugsweise in Wasser oder in einem Gemisch aus Wasser und einem mit Wasser leicht mischbaren organischen Lösungsmittel, wie z. B. Aceton, Dioxan, Acetonitril, Dimethylformamid, Dimethylsulfoxid oder Äthanol durchgeführt. Die Reaktionstemperatur liegt im allgemeinen im Bereich von etwa 10 bis etwa 100°C, vorzugsweise zwischen 20 und 80°C. Die Basenkomponente wird in Mengen zugegeben, die zwischen etwa äquimolaren Mengen und einem bis zu etwa 15-fachen Überschuß liegt. Der Austausch des Restes $R^9$ wird durch Anwesenheit von Neutralsalzionen, vorzugsweise von Jodid- oder Thiocyanationen im Reaktionsmedium erleichtert. Insbesondere werden etwa 10 bis etwa 80 Äquivalente Kaliumjodid, Natriumjodid, Kaliumthiocyanat oder Natriumthiocyanat zugeben. Die Reaktion wird vorteilhaft in der Nähe des Neutralpunktes, vorzugsweise bei einem pH-Wert im Bereich von etwa 5 bis etwa 8 durchgeführt.

Liegt die Gruppe $R^8$ als geschützte Aminofunktion vor, so eignen sich als Aminoschutzgruppen z. B. gegebenenfalls substituiertes Alkyl, wie beispielsweise tert.-Butyl, tert.-Amyl, Benzyl, p-Methoxybenzyl, Trityl, Benzhydryl, vorzugsweise Trityl; Trialkylsilyl, wie beispielsweise Trimethylsilyl; gegebenenfalls substituiertes aliphatisches Acyl, wie z. B. Formyl, Chloracetyl, Bromacetyl, Trichloracetyl und Trifluoracetyl, vorzugsweise Formyl; oder gegebenenfalls substituiertes Alkoxycarbonyl wie beispielsweise Trichloräthoxycarbonyl, Benzyloxycarbonyl oder tert.-Butyloxycarbonyl, vorzugsweise tert.-Butyloxycarbonyl und Benzyloxycarbonyl; oder Dimethylaminomethylen.

Die Schutzgruppe kann nach der Austauschreaktion in an sich bekannter Weise abgespalten werden, z. B. die Tritylgruppe mittels einer Carbonsäure, wie z. B. Essigsäure, Trifluoressigsäure, Ameisensäure oder die Benzyloxycarbonylgruppe hydrogenolytisch.

Die Reaktionsprodukte der Formel I können aus der Reaktionsmischung in üblicher Weise, z. B. durch Gefriertrocknen der Wasserphase, Chromatographie oder auch durch Ausfällen als schwerlösliches Salz, beispielsweise als Hydrojodid- oder Hydrothiocyanatsalz, isoliert werden.

Die nucleophile Austauschreaktion an Verbindungen der allgemeinen Formel II, kann auch so erfolgen, daß die Reaktion in Gegenwart der den Resten A entsprechenden Base, wie z. B. Chinolin oder Isochinolin und von Trimethyljodsilan vorgenommen wird. Diese Variante der Austauschreaktion wird vorteilhafterweise so durchgeführt, daß zu einem Gemisch der Verbindung II und der Base in einem geeigneten Lösungsmittel Trimethyljodsilan zugegeben wird. Es kann aber auch so verfahren werden daß die Verbindung II zuerst mit Trimethyljodsilan nach den im folgenden genannten Reaktionsbedingungen zur Reaktion gebracht wird und sodann die Base zugegeben wird.

Geeignete Lösungsmittel sind chlorierte Kohlenwasserstoffe, wie Methylenchlorid, Chloroform Dichloräthan, Trichloräthan, Tetrachlorkohlenstoff, oder Niederalkylnitrile, wie Acetonitril oder Propionitril.

Die Base wird in mindestens stöchiometrischer Menge bis zu einem zwanzigfachen Überschuß zugegeben, vorzugsweise wird mit einem fünf bis fünfzehnfachen Überschuß gearbeitet.

Trimethyljodsilan wird ebenfalls in mindestens stöchiometrischer Menge bis zu einem zwanzigfachen Überschuß, vorzugsweise in einem fünf bis fünfzehnfachen Überschuß zugegeben.

Die Reaktion wird bei Temperaturen zwischen -5° und +100°, vorzugsweise zwischen 10° und 80°C ausgeführt.

Die Reaktionsprodukte der Formel I können nach Hydrolyse der Reaktionsmischung durch Zugabe von Wasser oder wässriger Mineralsäuren, z. B. verdünnter HCl, HBr, HJ oder $H_2SO_4$ aus der wässrigen phase in üblicher Weise, z. B. durch Gefriertrocknen der Wasserphase, Chromatographie und dergleichen isoliert werden. Vorzugsweise werden die polaren Reaktionsprodukte aus der wässrigen Lösung in Form eines schwerlöslichen Salzes, beispielsweise nach Zugabe von KSCN oder KJ als Hydrothiocyanat- oder Hydrojodidsalz ausgefällt.

Für den Fall, daß $R^9$ für eine Carbamoyloxygruppe steht, wird die Austauschreaktion analog durchgeführt.

Steht $R^9$ für Halogen, insbesondere Brom oder Jod, so erfolgt der Austausch in literaturbekannter Weise.

Liegt die Verbindung II dabei in Form eines reaktionsfähigen Derivates vor, so kommen beispielsweise Silylderivate in Betracht, die bei der Umsetzung von Verbindungen der allgemeinen Formel II mit einer Silylverbindung gebildet werden, wie z. B. Trimethylchlorsilan, Bis-(trimethylsilyl)acetamid oder Bis(trimethylsilyl)trifluoroacetamid. In diesem Fall wird die Umsetzung zweckmäßig in Gegenwart eines inerten Lösungsmittels, wie Methylenchlorid oder Acetonitril vorgenommen.

Die Acylierung der Verbindungen der allgemeinen Formel III oder von deren Additionssalzen, beispielsweise mit Chlorwasserstoffsäure, Bromwasserstoffsäure, Salpetersäure, Schwefelsäure, Phosphorsäure, oder einer organischen Säure, wie z. B. Methansulfonsäure, p-Toluolsulfonsäure oder Maleinsäure, wird mit Carbonsäuren der allgemeinen-Formel IV oder mit einem reaktionsfähigen Derivat einer solchen Säure durchgeführt. In manchen Fällen ist es dabei von Vorteil, die 2-Aminogruppe in den Verbindungen der allgemeinen Formel IV vor der Umsetzung zu schützen. Als Aminoschutzgruppen eignen sich die vorstehend für $R_8$ beschriebenen Schutzgruppen. Die Schutzgruppe kann nach der Acylierung in an sich bekannter Weise abgespalten werden, z. B. die Tritylgruppe mittels einer Carbonsäure, wie z. B. Ameisensäure oder Trifluoressigsäure, oder die Chloracetylgruppe mittels Thioharnstoff. Werden die Carbonsäuren der allgemeinen Formel IV sowie ihre an der Aminogruppe geschützten Derivate selbst als Acylierungsmittel eingesetzt, so wird zweckmäßig in Gegenwart eines Kondensationsmittels, beipsielsweise eines Carbodiimids, wie beispielsweise N,N'-Dicyclohexylcarbodiimid gearbeitet.

Die Aktivierung der Carbonsäuren der allgemeinen Formel IV kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie in der Deutschen Patentschrift 2 804 040 beschrieben wird.

Als aktivierte Derivate der Carbonsäuren der allgemeinen Formel IV eignen sich insbesondere auch Halogenide, vorzugsweise Chloride, die in an sich bekannter Weise durch Behandlung mit Halogenierungsmitteln, wie z. B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel IV eignen sich ferner die Anhydride und gemischten Anhydride, Azide, aktivierten Ester und Thioester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol und 8-Chlor-1-hydroxybenzotriazol und 2-Mercaptobenzthiazol. Als gemischte

Anhydride sind besonders geeignet solche mit niederen Alkansäuren, wie z. B. Essigsäure, und besonders bevorzugt solche mit substituierten Essigsäuren, wie z. B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel IV, in denen die Aminogruppe geschützt ist, mit Chlorameisensäurebenzylester, -p-nitrobenzylester, -isobutylester, -ethylester oder -allylester gewinnt. Die aktivierten Derivate können als isolierte Substanzen, aber auch in situ umgesetzt werden.

Im allgemeinen erfolgt die Umsetzung der Cephemderivate der allgemeinen Formel III mit einer Carbonsäure der allgemeinen Formel IV oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z. B. Diäthyläther, vorzugsweise Tetrahydrofuran und Dioxan; Ketone, wie vorzugsweise Aceton und Butanon; Amide, wie vorzugsweise Dimethylformamid und Dimethylacetamid, oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel III mit einem in situ erzeugten aktivierten Derivat einer Carbonsäure der Formel IV umgesetzt wird.

Die Umsetzung von Cephemvorbindungen der Formel III mit Carbonsäuren der Formel IV bzw. deren aktivierten Derivaten kann in einem Temperaturbereich von etwa -80 bis etwa +80° C, vorzugsweise zwischen -30 und +50° C, insbesondere jedoch zwischen etwa -20° C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z. B. Triäthylamin, Dimethylanilin oder Pyridin; anorganische Basen, wie z. B. Kaliumcarbonat oder Natriumcarbonat; Alkylenoxide, wie z. B. Propylenoxid. Auch die Anwesenheit eines Katalysators, wie z. B. von Dimethylaminopyridin kann gegebenenfalls von Vorteil sein.

Liegt in den Verbindungen der allgemeinen Formel III die Aminogruppe in Form eines reaktionsfähigen Derivats vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel III mit einer Silylverbindung gebildet werden, wie z. B. Trimethylchlorsilan oder Bis-(trimethylsilyl)acetamid. Wird die Umsetzung mit einer solchen, an der Aminogruppe aktivierten Verbindung durchgeführt, so ist es zweckmäßig, die Reaktion in einem inerten Lösungsmittel, wie z. B. Methylenchlorid, Tetrahydrofuran oder Dimethylformamid durchzuführen.

Als physiologisch verträgliche Säureadditionssalze der Verbindungen der allgemeinen Formel I seien beispielsweise erwähnt solche mit Chlorwasserstoffsäure, Bromwasserstoffsäure, Salpetersäure, Phosphorsäure, Schwefelsäure oder organischen Säuren, wie z. B. Methansulfonsäure, p-Toluolsulfonsäure oder Maleinsäure.

Die Verbindungen der allgemeinen Formel III können in an sich bekannter Weise, beispielsweise aus der 7-Aminocephalosporansäure oder an der Aminogruppe geschützten 7-Aminocephalosporansäure auf dieselbe Weise erhalten werden, wie sie vorstehend für den nucleophilen Austausch von $R^9$ beschrieben wurde.

Die Verbindungen der allgemeinen Formel IV sowie die den Resten A entsprechenden Chinolin- und Isochinolinderivate sind literaturbekannt oder nach literaturbekannten Verfahren herstellbar.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase- und cephalosporinase-bildende Bakterien sind die Verbindungen der Formel I unerwartet gut wirksam. Da sie zudem günstige toxikologische und pharmakologische Eigenschaften zeigen, stellen sie wertvolle Chemtherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I und ihre physiologisch verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel I mit einem oder mehreren pharmakologisch verträglichen Trägerstoffen oder Verdünnungsmittel, wie z. B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder einer zur parenteralen Applikation geeigneten Suspension oder Lösung.

Als Träger- oder Verdünnungmittel seien beispielsweise erwähnt Traganth, Milchzucker, Talkum, Agar-Agar, Polyglykole, Äthanol und Wasser. Puffersubstanzen sind beispielsweise organische Verbindungen wie z. B. N/N'-Dibenzyläthylendiamin, Diäthanolamin, Äthylendiamin, N-Methylglucamin, N-Benzylphenäthylamin, Diäthylamin, Tris(hydroxymethyl)aminomethan, oder anorganische Verbindungen, wie z. B. Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen

Säureadditionssalze liegen bei etwa 0,4 bis 20 g/Tag, vorzugsweise bei 0,5 bis 4 g/Tag für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg vorzugsweise von etwa 100 bis 500 mg enthalten kann.

Cephemverbindungen, die in 3-Stellung des Cephemrings eine durch einen nucleophilen Rest substituierte Methylgruppe tragen, werden bereits in der DE-OS-2 716 707 beansprucht. Es war jedoch nicht zu erwarten, daß die erfindungsgemäß erhaltenen Verbindungen sich gegenüber den in der DE-OS-2 716 707 erhaltenen Verbindungen durch eine unerwartete Überlegenheit ihrer antibakteriellen Eigenschaften auszeichnen würden.

Aus den U.S.-Patentschriften 4 450 270 und 4 278 793 sind weiterhin antimikrobiell wirksame Cephemderivate bekannt, die eine 2-Aminothiazol-4-yl-Gruppe und einen gegebenenfalls substituierten Chinoliniummethyl- oder Isochinoliniummethylrest als Substituenten in 3-Stellung des Cephemrings tragen.

Die folgenden Ausführungsbeispiele für erfindungsgemäß herstellbare syn-Verbindungen dienen zur weiteren Erläuterung der Erfindung.

**Beispiel 1**

7-[2-(2-Aminothiazol-4-yl)-2-syn-difluormethoxyiminoacetamido]-3-isochinoliniomethyl-ceph-3-em-4-carboxylat

**Lösung A:**

0.53 g 2-(2-Aminothiazol-4-yl)-2-syn-difluormethoxyimioessigsäure, 0.30 g 1-Hydroxybenzotriazolhydrat und 0.41 g Dicyclohexylcarbodiimid werden in 25 ml N,N-Dimethylformamid (DMF) suspendiert, 2 Stunden bei Raumtemperatur gerührt und vom Dicyclohexylharnstoff abfiltriert.

**Lösung B:**

0.68 g 7-Amino-3-jodmethyl-ceph-3-em-4-carbonsäure werden in 40 ml DMF suspendiert und nach Zugabe von 0.65 g Isochinolin 4 Stunden bei Raumtemperatur gerührt. Unter Eiskühlung wird Lösung A in Lösung B getropft und über Nacht bei Raumtemperatur gerührt. Die Lösung wird im Vakuum eingeengt, in wenig Wasser unter Zusatz von Natriumbicarbonat bis zum pH = 6 gelöst und an Kieselgel (Säule® Lobar-B, Fa. Merck) mit Aceton/Wasser (3 : 1) chromatographiert. Durch Gefriertrocknen der Produktfraktionen erhält man 0.09 g farblosen, amorphen Feststoff.

$^1$H-NMR ($CF_3CO_2D$): δ = 3.40 und 3.90 ($AB_q$, J = 18Hz, 2H, $SCH_2$), 5.30 - 6.42 (m, 4H, $CH_2N^{\oplus}$ und 2 Lactam-H), 6.70 (t, J = 72Hz, 1H, $CHF_2$), 7.45 (s, 1 Thiazol-H), 8.0 - 8.8 (m, 6 Isochinolin-H), 9.80 ppm (bs, 1 Isochinolin-H).

In analoger Weise wie in Beispiel 1 beschreiben, werden die nachfolgend aufgeführten Verbindungen erhalten, die der allgemeinen Formel I mit $R^2$ = Wasserstoff entsprechend und als Reste $R^1$, $R^3$ und A die in Tabelle 1 angegebenen Substituenten tragen.

**Tabelle 1**

| Beispiel | $R^1$ | $R^3$ | A | $^1$H-NMR in $CF_3CO_2D$: δ (ppm) = |
|---|---|---|---|---|
| 2 | H | $CH_2CF_3$ | 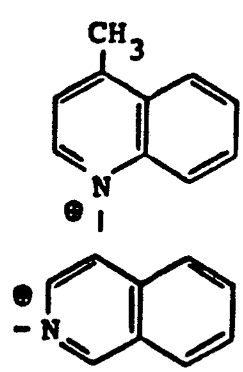 | 3.13 (s, 3H Lepidin-$CH_3$), 3.15 - 3.86 (AB, J = 18Hz, 2H $SCH_2$), 4.69 (br. q, J = 8Hz, 2H, $\underline{CH_2}$-$CF_3$), 5.26-6.68 (m, 4H, $CH_2N^{\oplus}$ und 2 Lactam-H), 7.39 (s, 1H, Thiazol), 7.76 - 8.79 (m, 5 Lepidin-H), 9.04 ("d", $J_{2,3}$ = 6Hz, Lepidin-2-H). |
| 3 | H | $CH_2CF_3$ | | 3.2 - 4.06 (AB, J = 19Hz, 2H, $SCH_2$), 4.66 (br. q, J=8Hz, 2H, -$\underline{CH_2}$-$CF_3$), 5.23 - 6.53 (m, 4H, $CH_2N^{\oplus}$ und 2 Lactam-H), 7.36 (s, 1H, Thiazol), 7.86 - 8.8 (m, 6 Isochinolin-H), 9.76 (mc, 1 Isochinolin-H). |

**Patentansprüche** für die Vertragsstaate: BE, CH, DE, FR, GB, IT, LI, LU, NL, SE

1. Cephemderivate der allgemeinen Formel I

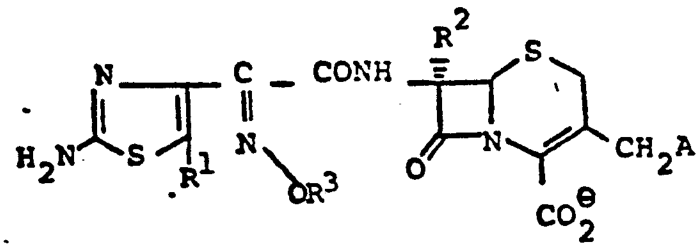

(I)

und deren physiologisch verträglichen Säureadditionssalze, worin bedeuten

$R^1$ Wasserstoff,
$R^2$ Wasserstoff,
$R^3$ gegebenenfalls durch Fluor substituiertes $C_1$-$C_4$-Alkyl,
A einen Chinolinium oder einen Isochinoliniumrest, der jeweils auch 1- bis 2-fach substituiert sein kann durch $C_1$-$C_4$-Alkyl,
und in der die $R^3O$-Gruppe in syn-Position steht

2. Verfahren zur Herstellung von Verbindungen der Formel I gemäß Anspruch 1 und ihre physiologisch verträglichen Säureadditionssalze, dadurch gekennzeichnet, daß man

a) eine Verbindung der allgemeinen Formel II

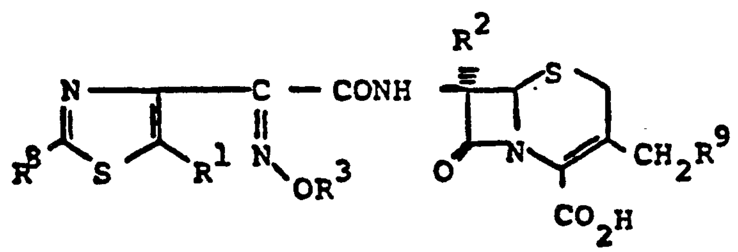

II

oder deren Salze oder ein reaktionsfähiges Derivat der Verbindung II worin $R^1$, $R^2$ und $R^3$ die obengenannte Bedeutung haben und $R^8$ eine Amino- oder eine geschützte Aminogruppe bedeutet und $R^9$ eine durch Chinolin, Isochinolin oder substituierte Chinoline oder Isochinoline, die den Resten A der Formel I entsprechen, austauschbare Gruppe bedeutet, mit Chinolin, Isochinolin oder einem ihrer Derivate umsetzt und

α) eine gegebenenfalls vorhandene Schutzgruppe abspaltet und
β) falls erforderlich, das erhaltene Produkt in ein physiologisch verträgliches Säureadditionssalz überführt,
oder

b) eine 7-Aminocephemverbindung der allgemeinen Formel III

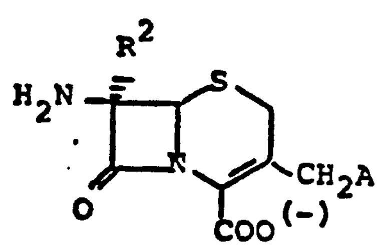

(III)

oder deren Säureadditionssalze, worin $R^2$ und A die obengenannte Bedeutung haben, wobei die Aminogruppe auch in Form eines reaktionsfähigen Derivats vorliegen kann, mit einer 2-(2-Aminothiazol-4-yl)-2-syn-oximinoessigsäure der allgemeinen Formel IV,

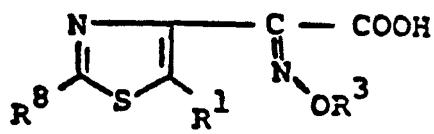

IV

worin $R^1$, $R^3$ und $R^8$ die obige Bedeutung besitzen, oder mit einem aktivierten Derivat dieser Verbindung umsetzt und

α) eine gegebenenfalls vorhandene Schutzgruppe abspaltet und
β) falls erforderlich, das erhaltene Produkt in ein physiologisch verträgliches Säureadditionssalz überführt.

3. Verfahren gemäß Anspruch 2, dadurch gekennzeichnet, daß der nucleophile Austausch des Substituenten $R^9$ in Gegenwart von Neutralsalzionen, insbesondere von Jodid- oder Thiocyanationen erfolgt.

4. Verfahren gemäß Anspruch 2, dadurch gekennzeichnet, daß der nucleophile Austausch des Substituenten $R^9$ in Gegenwart der dem Rest A zugrundeliegenden Base und von Trimethyljodsilan erfolgt.

5. Gegen bakterielle Infektionen wirksame pharmazeutische Präparate, gekennzeichnet, durch einen Gehalt an Cephemderivaten der allgemeinen Formel I gemäß Anspruch 1.

6. Verfahren zur Herstellung von gegen bakterielle Infektionen wirksamen pharmazeutischen Präparaten, dadurch gekennzeichnet, daß ein Cephemderivat der allgemeinen Formel I gemäß Anspruch 1, gegebenenfalls mit pharmazeutisch üblichen Trägerstoffen, Verdünnungsmitteln oder Puffersubstanzen in eine pharmazeutisch geeignete Verabreichungsform gebracht wird.

7. Verwendung von Cephemderivaten der allgemeinen Formel I gemäß Anspruch 1 zur Bekämpfung bakterieller Infektionen.

8. Cephemderivate gemäß Anspruch 1, dadurch gekennzeichnet, daß in der allgemeinen Formel I $R^1$ für Wasserstoff, $R^2$ für Wasserstoff, $R^3$ für $C_1$-$C_4$-Alkyl und A für Chinolin oder Isochinolin steht.

9. Cephemderivate gemäß Anspruch 8, dadurch gekennzeichnet, daß die $C_1$-$C_4$-Alkylgruppe für Methyl oder Äthyl steht.

**Patentansprüche** für der Vertragsstaat: AT

1. Verfahren zur Herstellung von Cephemderivaten der allgemeinen Formel I

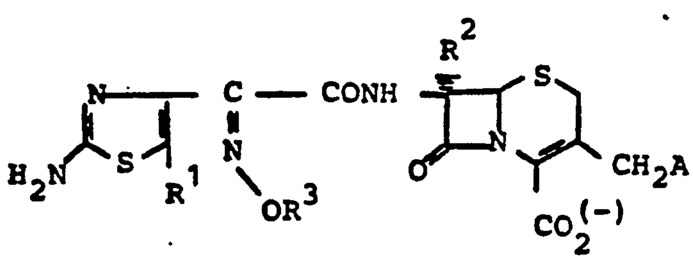

(I)

und ihrer physiologisch verträglichen Säureadditionssalze, worin bedeuten

$R^1$ Wasserstoff,
$R^2$ Wasserstoff,
$R^3$ gegebenenfalls durch Fluor substituiertes $C_1$-$C_4$-Alkyl,
A einen Chinolinium oder einen Isochinoliniumrest, der jeweils auch ein- oder zweifach substituiert sein kann durch $C_1$-$C_4$-Alkyl,

und in der die $R^3O$-Gruppe in syn-Position steht, dadurch gekennzeichnet, daß man

a) eine Verbindung der allgemeinen Formel II

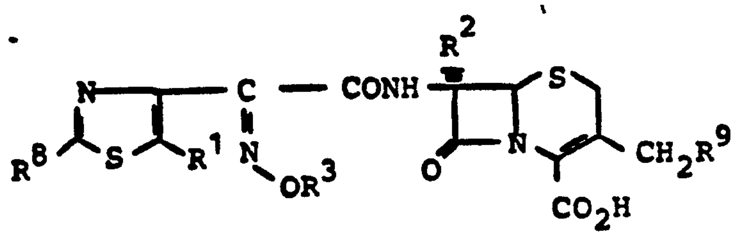

(II)

oder deren Salze oder ein reaktionsfähiges Derivat der Verbindung II worin $R^1$, $R^2$ und $R^3$ die obengenannte Bedeutung haben und $R^8$ eine Amino- oder eine geschützte Aminogruppe bedeutet und $R^9$ eine durch Chinolin, Isochinolin oder substituierte Chinoline oder Isochinoline, die den Resten A der Formel I entsprechen, austauschbare Gruppe bedeutet, mit Chinolin, Isochinolin oder einem ihrer Derivate umsetzt und

α) eine gegebenenfalls vorhandene Schutzgruppe abspaltet und
β) falls erforderlich, das erhaltene Produkt in ein physiologisch verträgliches Säureadditionssalz überführt, oder

b) eine 7-Aminocephemverbindung der allgemeinen Formel III

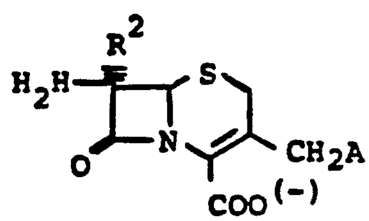

(III)

oder deren Säureadditionssalze, worin $R^2$ und A die obengenannte Bedeutung haben, wobei die Aminogruppe auch in Form eines reaktionsfähigen Derivats vorliegen kann, mit einer 2-(2-Aminothiazol-4-yl)-2-syn-oximinoessigsäure der allgemeinen Formel IV,

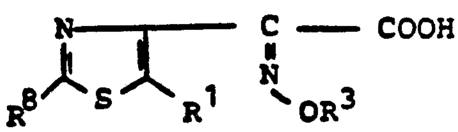

(IV)

worin $R^1$, $R^3$ und $R^8$ die obige Bedeutung besitzen, oder mit einem aktivierten Derivat dieser Verbindung umsetzt und

α) eine gegebenenfalls vorhandene Schutzgruppe abspaltet und
β) falls erforderlich, das erhaltene Produkt in ein physiologisch verträgliches Säureadditionssalz überführt.

2. Verfahren gemäß Anspruch 1, dadurch gekennzeichnet, daß der nucleophile Austausch des Substituenten $R^9$ in Gegenwart von Neutralsalzionen, insbesondere von Jodid- oder Thiocyanationen erfolgt.

3. Verfahren gemäß Anspruch 1, dadurch gekennzeichnet, daß der nucleophile Austausch des Substituenten $R^9$ in Gegenwart der dem Rest A zugrundeliegenden Base und von Trimethyljodsilan erfolgt.

4. Verfahren zur Herstellung von gegen bakterielle Infektionen wirksamen pharmazeutischen Präparaten, dadurch gekennzeichnet, daß ein Cephemderivat der allgemeinen Formel I gemäß Anspruch 1 gegebenenfalls mit pharmazeutisch üblichen Trägerstoffen, Verdünnungsmitteln oder Puffersubstanzen in eine pharmazeutisch geeignete Verabreichungsform gebracht wird.

5. Verfahren gemäß Anspruch 1, dadurch gekennzeichnet, daß in der allgemeinen Formel I gemäß Anspruch 1 $R^1$ für Wasserstoff, $R^2$ für Wasserstoff, $R^3$ für $C_1$-$C_4$-Alkyl und A für Chinolin oder Isochinolin steht.

6. Verfahren gemäß Anspruch 5, dadurch gekennzeichnet, daß die $C_1$-$C_4$-Alkylgruppe für Methyl oder Äthyl steht.

**Claims** for the contracting states: BE, CH, DE, FR, GB, IT, LI, LU, NL, SE

1. Cephem derivatives of the general formula

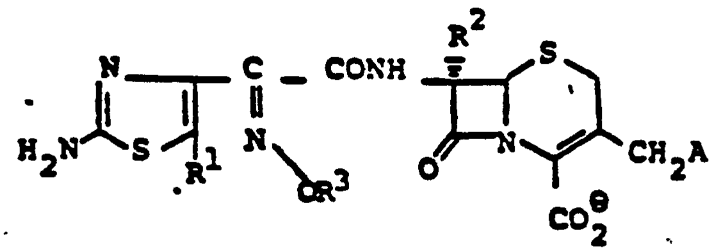

(I)

and physiologically acceptable acid addition salts thereof in which

$R^1$ denotes hydrogen,
$R^2$ denotes hydrogen,
$R^3$ denotes $C_1$-$C_4$-alkyl optionally substituted by fluorine,
A denotes a quinolinium or an isoquinolinium radical, each of which can also be substituted once or twice by $C_1$-$C_4$-alkyl

and in which the $R^3O$ group is in the syn position.

2. A process for the preparation of compounds of the formula I as claimed in claim 1 and their physiologically acceptable acid addition salts, which comprises

a) reacting a compound of the general formula II

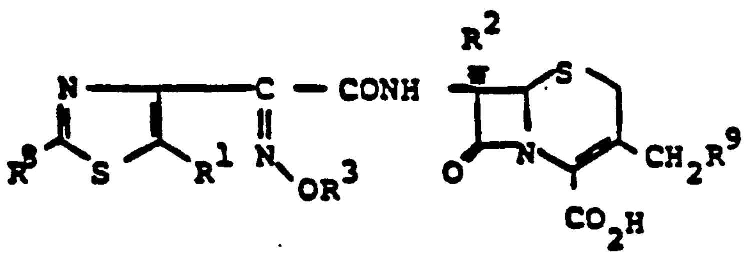

II

or salts thereof or a reactive derivative of the compound II in which $R^1$, $R^2$ and $R^3$ have the abovementioned meaning and $R^8$ denotes an amino group or a protected amino group and $R^9$ denotes a group which can be replaced by quinoline, isoquinoline or substituted quinolines or isoquinolines corresponding to the radicals A of the formula I, with quinoline, isoquinoline or one of their derivatives, and

α) splitting off a protective group which is present where appropriate and
β) if necessary, converting the resulting product into a physiologically acceptable acid addition salt, or

b) reacting a 7-aminocephem compound of the general formula III

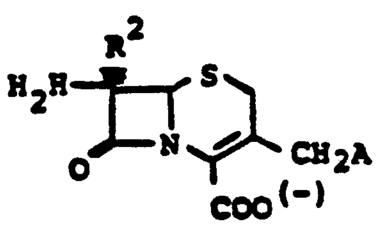

(III)

or acid addition salts thereof in which $R^2$ and A have the abovementioned meaning, it also being possible for the amino group to be present in the form of a reactive derivative, with a 2-(2-aminothiazol-4-yl)-2-syn-oximinoacetic acid of the general formula IV

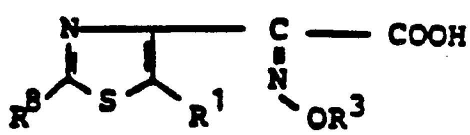

(IV)

in which $R^1$, $R^3$ and $R^8$ have the above meaning, or with an activated derivative of this compound, and

α) splitting off a protective group which is present where appropriate and
β) if necessary, converting the resulting product into a physiologically acceptable acid addition salt.

3. The process as claimed in claim 2, wherein the nucleophilic replacement of the substituent $R^9$ is effected in the presence of neutral salt ions, in particular of iodide or thiocyanate ions.

4. The process as claimed in claim 2, wherein the nucleophilic replacement of the substituent $R^9$ is effected in the presence of the base from which the radical A is derived, and of trimethyliodosilane.

5. Pharmaceutical products which are effective against bacterial infections and contain cephem derivatives of the general formula I as claimed in claim 1.

6. A process for the preparation of pharmaceutical products which are effective against bacterial infections, which comprises bringing a cephem derivative of the general formula I as claimed in claim 1 into a pharmaceutically suitable administration form, if appropriate together with pharmaceutically customary excipients, diluents or buffer substances.

7. The use of cephem derivatives of the general formula I as claimed in claim 1 for combating bacterial infections.

8. Cephem derivatives as claimed in claim 1, wherein, in the general formula I, $R^1$ represents hydrogen, $R^2$ represents hydrogen, $R^3$ represents $C_1$-$C_4$-alkyl and A represents quinoline or isoquinoline.

9. Cephem derivatives as claimed in claim 8, wherein the $C_1$-$C_4$-alkyl group represents methyl or ethyl.

**Claims** for the contracting state: AT

1. A process for the preparation of cephem derivatives of the general formula I

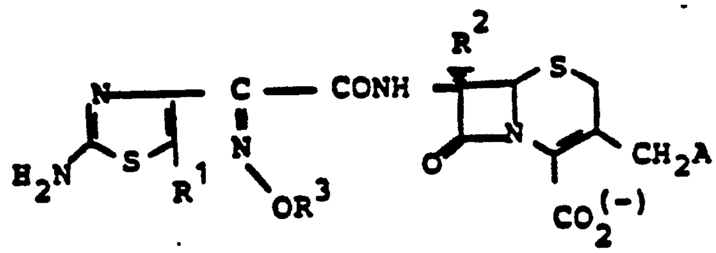

(I)

and physiologically acceptable acid addition salts thereof in which

$R^1$ denotes hydrogen,
$R^2$ denotes hydrogen,
$R^3$ denotes $C_1$-$C_4$-alkyl optionally substituted by fluorine,
A denotes a quinolinium or an isoquinolinium radical, each of which can also be substituted once or twice by $C_1$-$C_4$-alkyl and in which the $R^3O$ group is in the syn position,

which comprises

a) reacting a compound of the general formula II

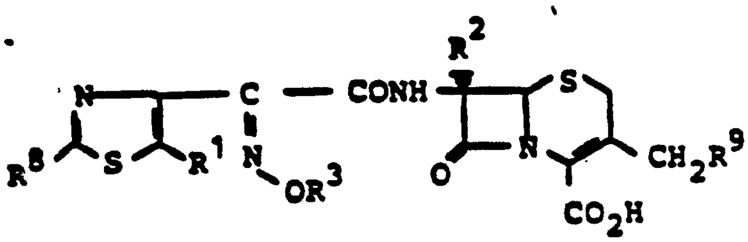

(II)

or salts thereof or a reactive derivative of the compound II in which $R^1$, $R^2$ and $R^3$ have the abovementioned meaning and $R^8$ denotes an amino group or a protected amino group and $R^9$ denotes a group which can be replaced by quinoline, isoquinoline or substituted quinolines or isoquinolines corresponding to the radicals A of the formula I, with quinoline, isoquinoline or one of their derivatives, and

α) splitting off a protective group which is present where appropriate and
β) if necessary, converting the resulting product into a physiologically acceptable acid addition salt, or

b) reacting a 7-aminocephem compound of the general formula III

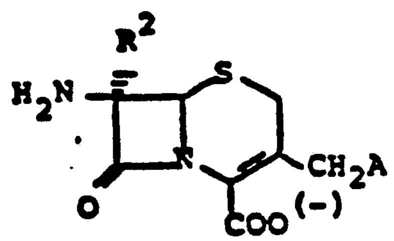

(III)

or acid addition salts thereof in which $R^2$ and A have the abovementioned meaning, it also being possible for the amino group to be present in the form of a reactive derivative, with a 2-(2-aminothiazol-4-yl)-2-syn-oximinoacetic acid of the general formula IV

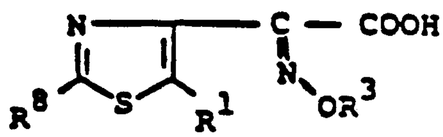

IV

in which $R^1$, $R^3$ and $R^8$ have the above meaning, or with an activated derivative of this compound, and

α) splitting off a protective group which is present where appropriate and
β) if necessary, converting the resulting product into a physiologically acceptable acid addition salt.

2. The process as claimed in claim 1, wherein the nucleophilic replacement of the substituent $R^9$ is effected in the presence of neutral salt ions, in particular of iodide or thiocyanate ions.

3. The process as claimed in claim 1, wherein the nucleophilic replacement of the substituent $R^9$ is effected in the presence of the base from which the radical A is derived, and of trimethyliodosilane.

4. A process for the preparation of pharmaceutical products which are effective against bacterial infections, which comprises bringing a cephem derivative of the general formula I as claimed in claim 1 into a pharmaceutically suitable administration form, if appropriate together with pharmaceutically customary excipients, diluents or buffer substances.

5. The process as claimed in claim 1, wherein, in the general formula I as claimed in claim 1, $R^1$ represents hydrogen, $R^2$ represents hydrogen, $R^3$ represents $C_1$-$C_4$-alkyl and A represents quinoline or isoquinoline.

6. The process as claimed in claim 5, wherein the $C_1$-$C_4$-alkyl group represents methyl or ethyl.

**Revendications** pour les Etats Contractants: BE, CH, DE, FR, GB, IT, LI, LU, NL, SE,

1. Derivés céphème de formule générale I

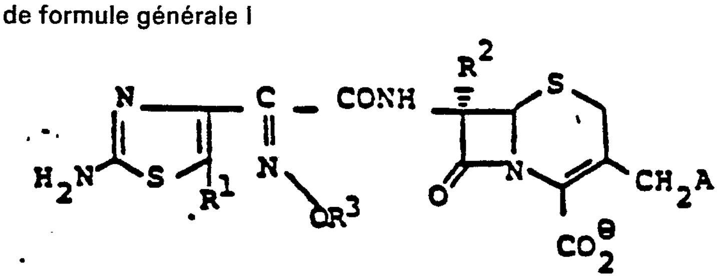

(I)

et sels d'addition de ceux-ci avec des acides physiologiquement acceptables, dans laquelle formule

$R^1$ représente un atome d'hydrogène;
$R^2$ représente un atome d'hydrogène;
$R^3$ représente un groupe alkyle en $C_1$-$C_4$ éventuellement substitué par des atomes de fluor;
A représente un reste quinoléinium ou isoquinoléinium qui peut dans chaque cas être également substitué une ou deux fois par un groupe alkyle en $C_1$-$C_4$;

et dans laquelle le groupe $OR^3$ est en position syn.

2. Procédé pour la préparation de composés de formule I selon la revendication 1 et de leurs sels d'addition avec des acides physiologiquement acceptables, caractérisé en ce que

a) on fait réagir un composé de formule générale II

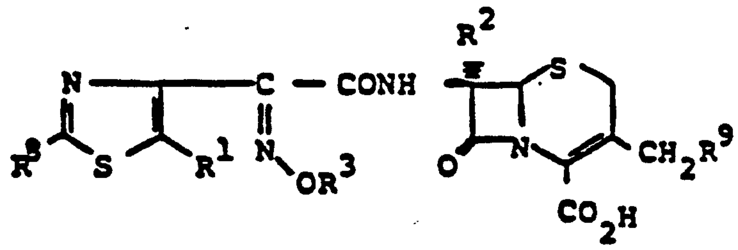

(II)

ou un de ses sels ou un dérivé réactif du composé II, dans lequel $R^1$, $R^2$ et $R^3$ ont les significations données plus haut, et $R^8$ représente un groupe amino ou un groupe amino protégé, et $R^9$ représente un groupe remplaçable par la quinoléine, l'isoquinoléine ou par des quinoleines ou isoquinoléines substituées qui correspondent aux restes A de la formule I,

avec la quinoléine, l'isoquinoléine ou un de leurs dérivés, et

α) on élimine un groupement protecteur éventuellement présent, et
β) si nécessaire, on convertit le produit obtenu en un sel d'addition avec un acide physiologiquement acceptable,

ou

b) on fait réagir un composé 7-aminocéphème de formule générale III

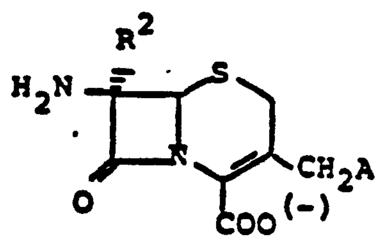

(III)

ou un de ses sels d'addition avec des acides, dans laquelle formule $R^2$ et A ont les significations données plus haut, le groupe amino pouvant également être présent sous forme d'un dérivé réactif, avec un acide 2-(2-aminothiazol -4-yl)-2-syn-oxyiminoacétique de formule générale IV

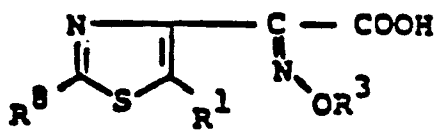

(IV)

dans laquelle $R^1$, $R^3$ et $R^8$ ont les significations données plus haut, ou avec un dérivé activé de ce composé; et

α) on élimine un groupement protecteur éventuellement présent, et
β) si nécessaire, on convertit le produit obtenu en un sel d'addition avec un acide physiologiquement acceptable.

3. Procédé selon la revendication 2, caractérisé en ce que l'on effectue le remplacement nucléophile du substituant $R^9$ en présence d'ions de sels neutres, en particulier d'ions iodure ou thiocyanate.

4. Procédé selon la revendication 2, caractérisé en ce que l'on effectue le remplacement nucléophile du substituant $R^9$ en présence de la base correspondant au reste A, et de triméthyliodosilane.

5. Compositions pharmaceutiques actives contre des infections bactériennes, caractérisées par une teneur en dérivés céphème de formule générale I selon la revendication 1.

6. Procédé pour la préparation de compositions pharmaceutiques actives contre des infections bactériennes, caractérisé en ce que l'on met sous une forme d'administration pharmaceutiquement appropriée un dérivé céphème de formule générale I selon la revendication 1, éventuellement avec des véhicules, diluants ou substances tampon pharmaceutiquement usuels.

7. Utilisation de dérivés céphème de formule générale I selon la revendication 1, pour la lutte contre des infections bactériennes.

8. Dérivés céphème selon la revendication 1, caractérisés en ce que, dans la formule générale I, $R^1$ représente un atome d'hydrogène, $R^2$ représente un atome d'hydrogène, $R^3$ représente un groupe alkyle en $C_1$-$C_4$ et A représente la quinoléine ou l'isoquinoléine.

9. Dérivés céphème selon la revendication 8, caractérisés en ce que le groupe alkyle en $C_1$-$C_4$ est un groupe méthyle ou éthyle.

**Revendications** pour l'Etat Contractant: AT

1. Procédé pour la préparation de dérivés céphème de formule générale I

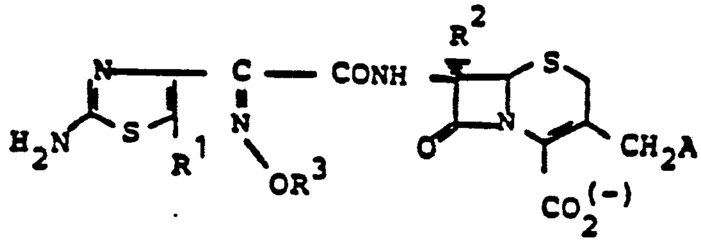

(I)

et de leurs sels d'addition avec des acides physiologiquement acceptables, dans laquelle formule

$R^1$ représente un atome d'hydrogène;
$R^2$ représente un atome d'hydrogène;
$R^3$ représente un groupe alkyle en $C_1$-$C_4$ éventuellement substitué par des atomes de fluor;
A représente un reste quinoléinium ou isoquinoléinium qui peut dans chaque cas être également substitué une ou deux fois par un groupe alkyle en $C_1$-$C_4$;
et dans laquelle le groupe $OR^3$ est en position syn, caractérisé en ce que

a) on fait réagir un composé de formule générale II

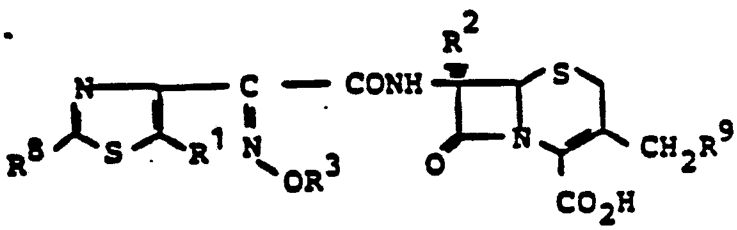

(II)

ou un de ses sels ou un dérivé réactif du composé II, dans lequel $R^1$, $R^2$ et $R^3$ ont les significations données plus haut, et $R^8$ représente un groupe amino ou un groupe amino protégé, et $R^9$ représente un groupe remplaçable par la quinoléine, l'isoquinoléine ou par des quinoléines ou isoquinoléines substituées qui correspondent aux restes A de la formule I,
avec la quinoléine, l'isoquinoléine ou un de leurs dérives, et

α) on élimine un groupement protecteur éventuellement présent, et
β) si nécessaire, on convertit le produit obtenu en un sel d'addition avec un acide physiologiquement acceptable,
ou

b) on fait réagir un composé 7-aminocéphème de formule générale III

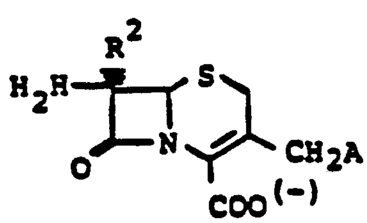

(III)

ou un de ses sels d'addition avec des acides, dans laquelle formule $R^2$ et A ont les significations données plus haut, le groupe amino pouvant également être présent sous forme d'un dérivé réactif, avec un acide 2-(2-aminothiazol -4-yl)-2-syn-oxyiminoacétique de formule générale IV

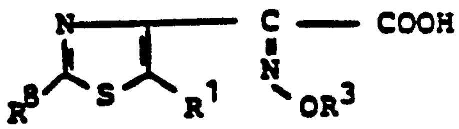

(IV)

dans laquelle $R^1$, $R^3$ et $R^8$ ont les significations données plus haut, ou avec un dérivé activé de ce composé; et

α) on élimine un groupement protecteur éventuellement présent, et

β) si nécessaire, on convertit le produit obtenu en un sel d'addition avec un acide physiologiquement acceptable.

2. Procédé selon la revendication 1, caractérisé en ce que l'on effectue le remplacement nucléophile du substituant $R^9$ en présence d'ions de sels neutres, en particulier d'ions iodure ou thiocyanate.

3. Procedé selon la revendication 1, caractérisé en ce que l'on effectue le remplacement nucléophile du substituant $R^9$ en présence de la base correspondant au reste A, et de triméthyliodosilane.

4. Procédé pour la préparation de compositions pharmaceutiques actives contre des infections bactériennes, caractérisées en ce que l'on met sous une forme d'administration pharmaceutiquement appropriée un dérivé céphème de formule generale I selon la revendication 1, éventuellement avec des véhicules, diluants ou substances tampon pharmaceutiquement usuels.

5. Procédé selon la revendication 1, caractérisé en ce que, dans la formule générale I selon la revendication 1, $R^1$ représente un atome d'hydrogène, $R^2$ représente un atome d'hydrogène, $R^3$ représente un groupe alkyle en $C_1$-$C_4$ et A représente la quinoléine ou l'isoquinoléine.

6. Procédé selon la revendication 5, caractérisé en ce que le groupe alkyle en $C_1$-$C_4$ est un groupe méthyle ou éthyle.